(19) **Europäisches Patentamt / European Patent Office / Office européen des brevets**

(11) **EP 4 323 071 B1**

(12) # EUROPEAN PATENT SPECIFICATION

(45) Date of publication and mention
of the grant of the patent:
**01.07.2026 Bulletin 2026/27**

(21) Application number: **22722833.5**

(22) Date of filing: **14.04.2022**

(51) International Patent Classification (IPC):
*A61Q 19/10* (2006.01)  *A61Q 19/00* (2006.01)
*A61K 8/44* (2006.01)  *A61K 8/60* (2006.01)
*A61K 8/73* (2006.01)  *A61K 8/365* (2006.01)
*C11D 1/66* (2006.01)  *C11D 1/90* (2006.01)
*C11D 3/20* (2006.01)  *C11D 3/22* (2006.01)

(52) Cooperative Patent Classification (CPC):
**A61Q 19/10; A61K 8/365; A61K 8/44; A61K 8/60;
A61K 8/604; A61K 8/73; A61Q 19/005;**
A61K 2800/30; A61K 2800/596

(86) International application number:
**PCT/EP2022/060049**

(87) International publication number:
**WO 2022/219133 (20.10.2022 Gazette 2022/42)**

(54) **CLEANSING COMPOSITION FOR FEMALE INTIMATE HYGIENE**

REINIGUNGSMITTEL FÜR DIE WEIBLICHE INTIMHYGIENE

CLEANSING COMPOSITION FOR FEMALE INTIMATE HYGIENECLEANSING COMPOSITION FOR FEMALE INTIMATE HYGIENE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.04.2021 IN 202121017757
07.06.2021 EP 21177978**

(43) Date of publication of application:
**21.02.2024 Bulletin 2024/08**

(73) Proprietors:
• **Unilever IP Holdings B.V.
6708 WH Wageningen (NL)**
Designated Contracting States:
**AL AT BE BG CH CZ DK EE ES FI FR GR HR HU IS
LI LT LU LV MC MK NL NO PL PT RO SE SI SK SM**
• **Unilever Global IP Limited
Wirral, Merseyside CH62 4ZD (GB)**
Designated Contracting States:
**CY DE GB IE IT MT RS TR**

(72) Inventors:
• **BHARADWAJ, Samarth
6708 WH Wageningen (NL)**
• **GUPTA, Sandeep
6708 WH Wageningen (NL)**
• **KAPOOR, Renu
6708 WH Wageningen (NL)**
• **MITRA, Rupak
6708 WH Wageningen (NL)**
• **NAIR, Rohini Sukumaran
6708 WH Wageningen (NL)**
• **YANG, Lin
6708 WH Wageningen (NL)**
• **YEKHE, Ashish Shrikant
6708 WH Wageningen (NL)**

(74) Representative: **Unilever Patent Group
Bronland 14
6708 WH Wageningen (NL)**

(56) References cited:
**EP-A1- 2 932 959    WO-A1-2006/015726
FR-A1- 3 040 624**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- **DATABASE GNPD [online] MINTEL; 8 December 2020 (2020-12-08), ANONYMOUS: "Intimate and Body Detergent", XP055864048, retrieved from https://www.gnpd.com/sinatra/recordpage/ 8322397/ Database accession no. 8322397**
- **DATABASE GNPD [online] MINTEL; 23 July 2020 (2020-07-23), ANONYMOUS: "Balancing Dandelion Feminine Gel", XP055892493, retrieved from https://www.gnpd.com/sinatra/ recordpage/7973111/ Database accession no. 7973111**

## EP 4 323 071 B1

**Description**

**Field of the invention**

The invention relates to a cleansing composition for female intimate hygiene, particularly to a composition that balances the microbiome of female intimate region.

**Background of the invention**

Our skin is the largest organ in the body, especially one which has the largest surface area. It forms the first line of defence against microorganisms which may invade the body. When the body is infected on the skin or systemically, traditional approach to such hygiene problems has been to treat the skin/ body with antimicrobial actives that reduce or kill the germs. Some bacteria that permanently reside on the skin (called skin commensal bacteria) do not actually cause infections, rather they are beneficial bacteria that protect the skin against pathogens. Several mechanisms have been proposed to explain the protection and some of the popular ones are: physically occupying space on skin to prevent colonization of pathogens;

producing metabolites that ward-off harmful pathogenic organisms; generating metabolites that strengthen the innate defence mechanisms to prevent infection by harmful pathogens; and providing other benefits such as maintaining skin pH and barrier function. This ensures that the microbiome is maintained in a healthy balanced state for long term hygiene and health.

Such an approach assumes greater significance in the context of cleansing compositions that are suitable for intimate areas of female body.

Compositions which are formulated for handwash and bodywash are not necessarily suitable for the sensitive body parts for females, particularly in the vaginal region of the female body. The tissues in the vaginal region are particularly sensitive to chemicals and are more likely to irritation.

The vagina is a dynamic ecosystem that harbours naturally-occurring bacteria primarily, Lactobacilli. However, a variety of other organisms, including some potential pathogens, may also be present at lower concentrations. Within the normally healthy female body, the action of Lactobacilli converts glycogen to lactic acid, whereby the lactic acid provides a physiological lowering of pH. The normal pH level of the vagina of a healthy woman of reproductive age is 3.8 to 4.2. This acidic pH level provides some degree of protection by making the vagina less hospitable for e.g. *E.coli and G.vaginalis. E.coli* are surrogates of those bacteria that cause UTI. However, if this healthy microbiome is disrupted, bacterial vaginosis could occur, leading to vaginal discomfort ranging from itching to a burning sensation and vaginal discharge.

WO20244898 A1 (Unilever) discloses topical compositions having thymol, terpineol or an analogue, for restoring diversity of microbiota of amenable skin to a level normally indicative of healthy skin.

WO2017055789 A1 (Reckitt) discloses feminine intimate hygiene formulation which delivers antibacterial action, whilst maintaining a healthy balance of the skin's natural flora, is non-drying to the skin, provides a moisturising benefit, and is suitable for everyday use while at the same time providing protection of the intimate area. The composition is said to provide antimicrobial benefit against fungi, gram positive and gram negative bacteria and which also provides mild cleansing to the skin including protection of the commensal microflora of the skin. The aqueous composition comprises 0.1 to 10 wt % betaines, 0.1 to 5.0 wt % lactic acid, 0.1 to 20 wt % polyhydric C2 to C6 alcohol, optionally upto 10 wt % alkyl polyethoxy carboxylate, upto 10 wt % alkyl polyethoxy amides, >8 wt % alkyl ether sulphate. The pH of the compositions is 3.8 to 4.5. The compositions have a total of 10 to 20 wt % surfactants and 0.1 to 10 wt % metal salt.

US20190262292 A1 (Reckitt) discloses intimate hygiene compositions having pH 3.5 to 5, comprising lactic acid or a salt and a ternary anionic constituent system. This system comprises a secondary alkane sulfonate compound(s), an N-acyl sarcosinate compound(s) and an aromatic hydrotrope compound(s) preferably a cumene sulfonate compound. The ternary anionic constituent system is said to boost the antimicrobial efficacy of the primary antimicrobial active against *S. aureus, E. coli, C. albicans and/or K. pneumoniae.*

EP1481666 A1 (Rottapharm, 2004) discloses compositions for rebalancing the vaginal ecosystem by reconstitution of an environment which favours the development of Lactobacilli. The composition comprises lactic acid for regulating the pH and a nutrient support comprising maltodextrin.

WO2019093060 (Gallinee) discloses solid cosmetic formulation having a base formulation including at least one syndet, at least one prebiotic and at least one postbiotic. Syndets include anionics like sodium lauryl sulfate, acyl isethionate, sodium alkylsulfonsuccinate, sodium cocoyl isethionate or disodium lauryl sulfosuccinate), cationics such as benzalkonium chloride, or amphoteric surfactants (such as, for example, cocamidopropyl betaine, coco ampho acetate or even diacetate), or even nonionic surfactants. Prebiotics are considered non-digestible fibers and can be classified according to their degree of polymerization into monosaccharides, disaccharides, or oligosaccharides. The term "postbiotic" is understood to mean a bacterial product or a metabolite of a probiotic organism which has a biological activity on the host. Lactic acid, acetic acid, butyric acid, propionic acid, fermentation products (products resulting from the transformation of certain organic materials, such as sugars, for example, under the action of enzymes secreted by microorganisms) and short chain fatty acids (comprising 1 to 6 carbon atoms) can be considered postbiotics.

US20190274937 A1 (Univ of Antwerp) discloses cosmetic cleaning compositions of pH less than 7, comprising at least one organic acid as preservative and at least one glycerol ester as surfactant. The compositions have typical characteristics of a soap without the negative effects on the natural microflora of the skin/vagina.

WO2006015726A1 (Henkel) discloses intimate washing compositions comprising lactic acid , nonionic surfactant and betaine.

We have determined that there is need for fast-acting cleansing compositions for female intimate hygiene but some of the compositions of prior art act indiscriminately against many microbes that inhabit the intimate area, therefore potentially disturbing the delicate balance of the microbiome.

Other compositions of prior art that are said to act selectively against microbes of a particular kind may not meet the requirement of being mild. Such compositions have the propensity to affect the permeability of the cells of the intimate area.

It has been determined that compositions in accordance with the present invention are able to meet the aforesaid contrasting technical requirements, thereby offering the option of fast-acting cleansing compositions for female intimate hygiene that do not disturb the delicate balance of the microbiome, are mild and do not affect permeability of the cells of the intimate area.

**Summary of the invention**

In accordance with a first aspect of the invention, disclosed is an aqueous sulphate-free cleansing composition for female intimate hygiene, said composition comprising:

a) 1 to 20 wt% of a non-soap surfactant system comprising combination of at least three non-soap sulphate-free surfactants selected from glycinates, betaines, alkyl polyglucosides and amphoacetates;
b) 0.05 to 4 wt% of a prebiotic capable of being broken down by lactobacilli to lactic acid; and,
c) 0.001 to 3 wt% lactic acid or a salt thereof added as a postbiotic;

wherein said composition is free of live bacteria including Lactobacilli.

In accordance with a second aspect disclosed is a non-therapeutic use of an aqueous composition as claimed in claim 1 for female intimate hygiene.

In accordance with a third aspect disclosed is a non-therapeutic method of providing female intimate hygiene comprising a step of contacting a female intimate region with said composition for a contact time of at least 30 seconds.

In accordance with a fourth aspect disclosed is an aqueous sulphate-free cleansing composition for use to provide female intimate hygiene, said composition comprising:

a) 1 to 20 wt% of a non-soap surfactant system comprising combination of at least three sulphate-free surfactants selected from glycinates, betaines, alkyl polyglucosides and amphoacetates;
b) 0.05 to 4 wt% of a prebiotic capable of being broken down by Lactobacilli to lactic acid; and,
c) 0.001 to 3 wt% lactic acid or a salt thereof added as a postbiotic;

wherein said composition is free of live bacteria including Lactobacilli.

In accordance with a fifth aspect disclosed is a packaged consumer product comprising an aqueous composition of the first aspect packaged in a plastic bottle made from 100% post-consumer recycled plastic.

**Detailed description of the invention**

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word comprising is intended to mean including but not necessarily consisting of or composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word about. Numerical ranges expressed in the format "from x to y" are understood to include x and y. When for a specific feature multiple preferred ranges are described in the format "from x to y", it is understood that all ranges combining the different endpoints are also contemplated.

"Skin" as used herein is meant to include skin on any part of the body (e.g. face, neck, chest, back, arms, underarms, hands, legs, buttocks and scalp). It is especially useful for protecting the skin of babies.

The term balancing means selectively reducing microbial count of at least one genus of a microbe considered to be harmful or of at least one genus of microbes that exhibit growth which is not representation of a normal healthy skin, while selectively increasing microbial count of at least one genus of microbes considered to be beneficial or of at least one genus of microbes whose numbers have reduced which is also not representation of a normal healthy skin.

Sometimes it is not easy to distinguish between beneficial and harmful microbes because it might vary depending on circumstances. Therefore, for dysbiotic conditions like acne or atopic dermatitis, balancing means reducing or increasing the count of those microbes that have significantly changed in a condition as against healthy state. Such change could either be an abnormal increase or an abnormal decrease in their count. A microbe can be described as a tiny living organism, such as bacterium, fungus, or virus. A microbiome or microbiota refers collectively to all the microbes on or in the human body. In other words, a microbiome is a community of microbes. A balanced microbiota containing diversity of organisms helps to maintain health and is essential for human development, immunity, health and wellbeing.

The vaginal ecosystem is primarily controlled by the predominance of Lactobacilli which, by producing lactic acid, guarantees conditions of environmental acidity able to regulate and balance the microbiome of the female intimate region such that proliferation of other unwanted micro-organisms is inhibited to at least some extent.

The normal circumstances do change for a variety of reasons, including inadequate hygiene and age. When such changes occur, the intimate region, especially the vaginal environment may have unwanted microbes such as E.coli and Gardenalia spp.

In extreme cases, the symptoms may indicate a state of "vaginosis" which may need medical treatment. However, simple day-to-day rituals such as the use of cosmetic cleansing compositions could go a long way to offer protection. However, the indiscriminate use of cleansing compositions may cause more harm. For example, the use of ordinary soap or bodywash compositions or shampoos to clean the female intimate region may affect the delicate microbiome and acidic environment of the region. Therefore, specialist cleansing compositions are necessary which may be made available in a variety of formats such as cleansing lotions, gels, wipes, foams, mousses etc.

The use of such specialist compositions is probably is the best way of restoring and rebalancing the vaginal ecosystem or in a broad sense, the ecosystem of the female intimate area such that the optimum conditions are created for the natural flora to develop and grow and at the same time to inhibit the growth of unwanted bacteria such as Gardenalia spp.

In accordance with the invention disclosed is an aqueous sulphate-free cleansing composition for female intimate hygiene, said composition comprising:

> a) 1 to 20 wt% of a non-soap surfactant system comprising combination of at least three sulphate-free surfactants selected from glycinates, betaines, alkyl polyglucosides and amphoacetates;
> b) 0.05 to 4 wt% of a prebiotic capable of being broken down by Lactobacilli to lactic acid; and,
> c) 0.001 to 3 wt% lactic acid or a salt thereof added as a postbiotic;

wherein said composition is free of live bacteria including Lactobacilli.

Preferably the non-soap surfactant system comprises combination of at least three sulphate-free surfactants.

The composition of the invention is aqueous. The term aqueous means that the composition comprises water. Preferably the compositions of the invention comprises 30 to 95 wt%, more preferably 40 to 90 wt% and further preferably 50 to 90 wt% water. The water forms a continous phase of the compostion.

The term sulphate-free means that the composition of the invention comprises not more 1 wt% of sulphated surfactants, in particular sodium lauryl sulphate and sodium lauryl ether sulphate.

All cleansing compositions usually contain one or more surfactants. The surfactants provide basic cleansing action. Some surfactants are usually used in combination with each other, thereby constituting a "surfactant system". A well-known example is that of SLES and CAPB. SLES is sodium lauryl ethoxy sulphate, an anionic surfactant and CAPB is coco amidopropyl betaine, a betaine type of surfactant.

The term non-soap means that the surfactant is not a soap. Soap means "fatty acid soap" or, more simply, "soap" is used here in its popular sense, i.e., salts of aliphatic alkane- or alkene monocarboxylic fatty acids preferably having 6 to 22 carbon atoms, and preferably 8 to 18 carbon atoms. Typical of the soap salts are alkali metal or alkanol ammonium salts of such fatty acids, although other metal salts thereof, e.g., magnesium salts, may also be employed. It is particularly prefered that the composition of the invention comprises less than 1 wt% soap.

The composition of the invention comprises 1 to 20 wt% of a non-soap surfactant system comprising combination of at least three sulphate-free surfactants selected from glycinates, betaines, alkyl polyglucosides and amphoacetates.

More preterably the composition comprises 4 to 15 wt%, further preterably 4 to 13 wt% surfactant. The term system implies a combination of surfactants and when the compositions of the invention comprises a combination, the cumulative amount is within the ranges defined herein. Alternatively, the composition comprises 1 to 20 wt% surfactants.

Alkanoyl glycinate(s) are prepared by reaction of $C_8$-$C_{18}$ fatty acid chloride with glycine in the presence of sodium, potassium, or ammonium hydroxide to form the corresponding $C_8$-$C_{18}$ alkanoyl glycinate.

Preferably, the amount of C12 to C18 chain length glycinate is predominant (greater than 50%, preferably greater than 60 %, more preferably 65 to 100%. More preferably 80 to 100% of total amount of C8, C10, and C12 to C18 chain length glycinate present.

It is preferred that the composition comprises 0.5 to 5 wt%, more preferably 0.5 to 3 wt% glycinates, particularly alkanoyl glycinates.

Betaines are amphoteric surfactants that include simple betaines of formula $R^1$-$N^+$-$(R^2)(R^3)CH_2CO_2^-$ and amido betaines of formula $R^1$ - $CONH(CH_2)_n$-$N^+$- $(R^2)(R^3)CH2CO2$, where n is 2 or 3. $R^1$ may in particular be a mixture of $C_{12}$ and $C_{14}$ alkyl groups derived from coconut oil so that at least half, preferably at least three quarters of the groups $R^1$ have 10 to 14 carbon atoms. $R^2$ and $R^3$ are preferably methyl.

A further possibility is that the betaine is a sulphobetaine of formula $R^1$-$N^+$- $(R^2)(R^3)$ $(CH_2)_3SO_3^-$ or $R^1$ - $CONH(CH_2)_m$-$N^+$- $(R^2)(R^3)$ $(CH_2)_3SO_3^-$, where m is 2 or 3, or variants of these in which - $(CH_2)_3$ $SO_3^-$ is replaced by -$CH_2C(OH)(H)CH_2SO_3^-$. Preferred betaines include alkyl amidopropylbetaine, particularly the betaine is cocoamidopropyl betaine.

It is preferred that the composition of the invention comprises 0.5 to 8 wt%, more preferably 1.5 to 8 wt% and most preferably 1.5 to to 7.5 wt% betaine.

Amphoacetates and diamphoacetates are also intended to be covered in possible zwitterionic and/or amphoteric compounds which may be used such as e.g., sodium lauroamphoacetate, sodium cocoamphoacetate, and blends thereof.

Preferred alkylpolyglycosides are of the formula wherein $R_2$ is selected from the group consisting of alkyl, alkylphenyl, hydroxyalkyl, hydroxyalkylphenyl, and mixtures thereof in which alkyl groups contain from about 10 to about 18, preferably from about 12 to about 14, carbon atoms; n is 0 to 3, preferably 2; t is from 0 to about 10, preferably 0; and x is from 1.3 to about 10, preferably from 1.3 to about 2.7. The glycosyl is preferably derived from glucose. To prepare these compounds, the alcohol or alkylpolyethoxy alcohol is formed first and then reacted with glucose, or a source of glucose, to form the glucoside (attachment at the 1-position). The additional glycosyl units can then be attached between their 1-position and the preceding glycosyl units 2-, 3-, 4- and/or 6-position, preferably predominantly the 2-position.

The alkyl polyglucoside(s) used in the context of the invention may or may not be polyalkoxylated.

According to a particular embodiment, they are chosen from the compounds having the following general formula: RO-(G), in which R' denotes a saturated or unsaturated, linear or branched hydrocarbon-based chain, comprising from 12 to 44 carbon atoms, the group G denotes a saccharide residue comprising from 5 to 6 carbon atoms and a is a number ranging from 1 to 10, preferably from 1 to 5 and even more preferentially a is equal to 1.

According to a particular embodiment, R denotes a branched alkyl radical comprising from 12 to 44 carbon atoms, preferably from 16 to 36 carbon atoms and better still from 18 to 22 carbon atoms.

The alkyl polyglucoside(s) may be chosen especially from the group comprising ethers or mixtures of ethers of fatty alcohols comprising from 12 to 44 carbon atoms and of glucose, maltose, sucrose, xylose or fructose, and ethers or mixtures of ethers of fatty alcohols comprising from 12 to 44 carbon atoms and of methylglucose. The fatty unit of the ethers may be chosen especially from cetyl, behenyl, arachidyl, stearyl, palmityl, myristyl, lauryl, hexadecanoyl and octyldodecyl units, and mixtures thereof such as cetearyl. Preferably, it is octyldodecyl.

According to a particular embodiment of the invention, the alkyl polyglucoside(s) are chosen from the compounds having the following formula: R'O-G, in which R' denotes a saturated branched alkyl radical comprising from 12 to 44 carbon atoms, preferably from 16 to 36 and better still from 18 to 22, and the group G denotes a saccharide residue comprising from 5 to 6 carbon atoms, preferably a xylose residue.

In particular, the alkyl polyglucoside is an octyldodecyl xyloside such as the product sold under the name Fluidanov 20 X by the company SEPPIC in the form of a mixture consisting of 75% octyldodecanol and 25% octyldodecyl xyloside.

It is preferred that pH of the composition of the invention is from 3.0 to 7.5. More preferably the pH is 3.2 to 7.2.

It is preferred that the composition of the invention is viscous but not too thick. Accordingly, it is preferred that the composition comprises a rheological additive such that viscosity of said composition is in the range of 2500 to 10000 cps. Preferably the viscosity is determined using a Brookfield V2 viscometer (spindle RTV5, 1 minute, 20rpm) at 30°C. The wt% of such an ingredient would vary depending on the type and the grade and all such routine adjustments are within the common general knowledge at the disposal of the skilled person. Examples of rheological additives which may be used include polyethylene glycol distearates, acylates, starch, derivatives of starch, waxes and other polymeric thickeners. It is preferred that the rheological additive is an acrylate polymer. Other suitable examples include water soluble or dispersible polymers include the carbohydrate gums such as cellulose gum, microcrystalline cellulose, cellulose gel, hydroxyethyl cellulose, hydroxypropyl cellulose, sodium carboxymethylcellulose, methyl cellulose, ethyl cellulose, guar gum, gum karaya, gum tragacanth, gum arabic, gum acacia, gum agar, xanthan gum and mixtures thereof; modified and nonmodified starch granules and pregelatinized cold water soluble starch; emulsion polymers such as Aculyn® 28, Aculyn® 22 or Carbopol ®Aqua SF1; cationic polymer such as modified polysaccharides including cationic guar available from Rhone Poulenc under the trade name Jaguar C13S, Jaguar C14S, Jaguar C17, or Jaguar C16; cationic modified cellulose such as UCARE Polymer JR 30 or JR 40 from Amerchol; N-Hance® 3000, N-Hance® 3196, N-Hance® GPX 215 or N-Hance® GPX 196 from Hercules; synthetic cationic polymer such as Merquat® 100, Merquat® 280, Merquat® 281 and Merquat® 550 sold by Nalco; cationic starches such as StaLok® 100, 200, 300 and 400 sold by Staley Inc.; cationic galactomannans such as Galactasol® 800 series by Henkel, Inc.; Quadrosoft® LM-200; and Polyquaternium-24. Also suitable are high molecular weight polyethylene glycols such as Polyox® WSR-205 (PEG 14M), Polyox® WSR-N-60K (PEG 45), and Polyox® WSR-301 (PEG 90M).

Compositions of the invention preferably include an inorganic electrolytes include metal chlorides (such as sodium chloride, potassium chloride, calcium chloride, magnesium chloride, zinc chloride, ferric chloride and aluminium chloride) and metal sulfates (such as sodium sulfate and magnesium sulfate, which are not sulphated surfactants). The inorganic electrolyte is separate from any inorganic electrolytes that may be present in the raw materials of the invention. The amount of inorganic electrolyte in compositions of the invention is preferably ranges from 0.5 to 5 %, more preferably from 1 to 5 %.

Prebiotic

Compositions of the invention comprise 0.05 to 4 wt% of a prebiotic capable of being broken down by Lactobacilli to lactic acid. It is preferred that the prebiotic is an oligosaccharide, preferably glycans, glucose, fructose, galactose, sucrose or lactose. Alternatively the prebiotic is a fructan, preferably inulin-type. Further alternatively it is a sugar alcohol, such as glycerol or lactitol. Further alternatively it is a complex polysaccharide, preferably β-glucans, cellulose or glycogen.

Preservative

Compositions of the invention preferably comprise a preservative. Preferably it is selected from phenoxyethanol, sodium benzoate or a mixture comprising or consisting of 4-isopropyl-3-methylphenol; at least one hydrophilic solvent having a log Po/w of 1.2 or below; and at least one lipophilic solvent having a log Po/w of above 1.2. Most preferably the preservative is Symguard CD, which comprises a combination of phenylpropanol, o-Cymen-5-ol and decylene glycol. Preferably the composition of the invention comprises 0.01 to 2% preservative.

Alternatively the composition may comprise a preservative selected from sodium salicylate, parabens, hydantoins, benzyl alcohol, benzoic acid, 1,2-alkanediols, iodopropynyl butylcarbamate (IPBC), 5-chloro-2-methyl-2H-isothiazol-3-one, 2-methyl-2H-isothiazol-3-one, caprylyl glycol or mixtures thereof.

Humectant

It is further preferred that the composition of the invention comprises 2 to 20 wt% humectant, more preferably 2.5 to 20wt%, further more preferably 3 to 15wt% and most preferably to 4 to 15wt% by weight of the composition. Preferably the humectant is propylene glycol, hexylene glycol, 1,4-dihydroxyhexane, 1,2,6-hexanetriol, sorbitol, butylene glycol, caprylyl glycol, propanediols, such as methyl propane diol, dipropylene glycol, triethylene glycol, glycerin (glycerol), polyethylene glycols, ethoxydiglycol, polyethylene sorbitol, glyceryl caprylate/caprate or a combination thereof.

Cationic Skin Conditioning Agents

Compositions according to the invention may also comprise a cationic skin feel agent or polymer, such as for example cationic celluloses. Cationic polymers are preferably used at levels as low as about 0.1 to 2 wt% up to levels as high as the solubility limit of the specific polymer, or preferably up to about 4 to 5 % by wt., provided that the solubility limit of the particular cationic polymer or blend thereof is not exceeded.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

A particularly suitable type of cationic polysaccharide polymer that can be used is a cationic guar gum derivative, such as guar hydroxypropyltrimonium chloride (Commercially available from Rhone-Poulenc in their JAGUAR trademark series). Examples are JAGUAR C13S, which has a low degree of substitution of the cationic groups and high viscosity, JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, JAGUAR 162 which is a high transparency, medium viscosity guar having a low degree of substitution, Jaguar Optima, which has a high degree of substitution and low molecular weight, and Jaguar Excel, which has a low degree of substitution and high viscosity. Particularly preferred cationic polymers are JAGUAR C13S, JAGUAR C15, JAGUAR C17 and JAGUAR C16 and JAGUAR C162, especially JAGUAR C13S, JAGUAR C-14/BFG, Jaguar Optima and Jaguar Excel. The JAGUAR C14/BFG material is the same molecule as JAGUAR C13, except that a glyoxal cross linker has replaced the boron. Other cationic skin feel agents known in the art may be used provided that they are compatible with the inventive formulation.

In addition, the inventive cleansing composition of the invention may include 0.001 to 15 wt% optional ingredients such as perfumes; sequestering agents, such as tetrasodium ethylenediaminetetraacetate (EDTA), EHDP or mixtures. The compositions may further comprise antimicrobials such as caprylyl glycol, 2-hydroxy-4,2', 4' trichlorodiphenylether (DP300); preservatives such as methylisothiazolinone / methylchloroisothiazolinone (Kathon, MIT), dimethyloldimethylhydantoin / iodopropynyl butylcarbamate (Glydant XL1000,) and sorbic acid.

Emollients

The term "emollient" is defined as a substance which softens or improves the elasticity, appearance, and youthfulness of the skin (stratum corneum) by either increasing its water content, adding, or replacing lipids and other skin nutrients; or both, and keeps it soft by retarding the decrease of its water content.

Hydrophobic emollients with weight average particle sizes below either 1000 or 500 microns in diameter are defined herein as "finely dispersed oils" and are preferably used at a minimum of 3, 2, 1 wt% upto 15 wt%.

These hydrophobic emollients include but are not limited to the following:

(a) silicone oils and modifications thereof such as linear and cyclic polydimethylsiloxanes; amino, alkyl, alkylaryl, and aryl silicone oils;

(b) fats and oils including natural fats and oils (triglycerides) such as jojoba, soybean, sunflower, safflower, algal, rice bran, avocado, almond, olive, sesame, persic, castor, coconut, mink oils; cacao fat; beef tallow, lard; hardened oils obtained by hydrogenating the aforementioned oils; and synthetic mono, di and triglycerides such as myristic acid glyceride and 2-ethylhexanoic acid glyceride;

(c) waxes such as carnauba, spermaceti, beeswax, lanolin, and derivatives thereof;

(d) hydrophobic plant extracts;

(e) hydrocarbons such as petrolatum, polybutene, liquid paraffins

(f) microcrystalline wax, ceresin, squalene, pristan and mineral oil;

(g) higher alcohols such as lauryl, cetyl, stearyl, oleyl, behenyl, cholesterol and 2-hexydecanol alcohol;

(h) esters such as cetyl octanoate, myristyl lactate, cetyl lactate, isopropyl myristate, myristyl myristate, isopropyl palmitate, isopropyl adipate, butyl stearate, decyl oleate, cholesterol isostearate, glycerol monostearate, glycerol distearate, glycerol tristearate, alkyl lactate, alkyl citrate and alkyl tartrate;

(i) essential oils and extracts thereof such as mentha, jasmine, camphor, white cedar, bitter orange peel, ryu, turpentine, cinnamon, bergamot, citrus unshiu, calamus, pine, lavender, bay, clove, hiba, eucalyptus, lemon, starflower, thyme, peppermint, rose, sage, sesame, ginger, basil, juniper, lemon grass, rosemary, rosewood, avocado, grape, grapeseed, myrrh, cucumber, watercress, calendula, elder flower, geranium, linden blossom, amaranth, seaweed, ginko, ginseng, carrot, guarana, tea tree, jojoba, comfrey, oatmeal, cocoa, neroli, vanilla, green tea, penny royal, aloe vera, menthol, cineole, eugenol, citral, citronelle, borneol, linalool, geraniol, evening primrose, camphor, thymol, spirantol, penene, limonene and terpenoid oils;

(j) mixtures of any of the foregoing components

Preferred emollients include petrolatum; natural wax; partially or fully hydrogenated triglyceride oils; and mixtures thereof. Preferred triglyceride oils include soybean oil or sunflower oil.

Optional active agents

Suitable active agents may be advantageously selected from antimicrobial and antifungal actives, vitamins, anti-skin atrophy and skin repair actives; skin barrier repair actives; non-steroidal cosmetic soothing actives; skin tightening agents; anti-itch ingredients; hair growth inhibitors; 5-alpha reductase inhibitors; desquamating enzyme enhancers; anti-glycation agents; topical anesthetics, or mixtures thereof.

Method and Use

In accordance with another aspect is disclosed non-therapeutic use of an aqueous composition of the first aspect for female intimate hygiene. Where the method or use is non-therapeutic in nature it preferably is for cosmetic applications. Alternatively, the use is therapeutic. By therapy is meant a method of bringing a body from a pathological state back into its normal healthy state or a method of preventing a pathological state.

It is preferred that the use leads to at least 5 -log reduction in the count of G.vaginalis. This species is associated with female intimate region and the vaginal microbiome. G.vaginalis is a facultatively anaerobic Gram-variable rod that is involved, together with many other bacteria, mostly anaerobic, in bacterial vaginosis in some women as a result of a disruption in the normal vaginal microflora. Therefore, it is preferable to reduce their number as much as possible. It is expected that the composition should produce quick results which could be ascertained through known methods. Usually the results are expressed in terms of log-reduction or percentage reduction in the microbes after such contact. A reasonably efficacious composition provides log-reduction to the extent of 3 log which translates to 99.9% reduction. More efficacious compostions provide 5-log which means 99.999 % reduction and the more potent ones can provide 7-log i.e. 99.99999 % reduction.

Further preferably the use leads to not more than 3-log reduction in the count of L.crispatus that also inhabit the female intimate region at a contact time of at least 30 seconds.

Lactobacillus crispatus is a common, rod-shaped species of genus Lactobacillus and is a hydrogen peroxide (H2O2) producing beneficial micro biota species located in both the vagina, through vaginal discharge, and the vertebrate gastrointestinal tract.

In accordance with another aspect is disclosed a non-therapeutic method of providing female intimate hygiene comprising a step of contacting a female intimate region with said composition for a contact time of at least 30 seconds. Preferably the count of G.vaginalis that inhabit the female intimate region is reduced by at least 5-log. More preferably the count of L.crispatus that inhabit the female intimate region is reduced by not more than 3-log.

Further in accordance with the invention is disclosed an aqueous sulphate-free cleansing composition for use to provide female intimate hygiene, said composition comprising:

  a) 1 to 20 wt% of a non-soap surfactant system comprising combination of at least three sulphate-free surfactants selected from glycinates, betaines, alkyl polyglucosides and amphoacetates;
  b) 0.05 to 4 wt% of a prebiotic capable of being broken down by Lactobacilli to lactic acid; and,
  c) 0.001 to 3 wt% lactic acid or a salt thereof added as a postbiotic;

wherein said composition is free of live bacteria including Lactobacilli.

In accordance with yet another aspect of the invention is disclosed a packaged consumer product comprising an aqueous composition of the first aspect packaged in a plastic bottle made from 100% post-consumer recycled plastic. The packaging could be a patch, bottle, bottle with dispenser, tube, roll-ball applicator, propellant driven aerosol device, squeeze container or lidded jar.

The invention will now be explained in detail with the following non-limiting examples.

**Examples**

Three cleansing compositions for female intimate hygiene in accordance with the invention were prepared. Details of the formulations are provided in Table 1.

Table 1

| Ingredients/wt% all at actuals | Reference | | |
|---|---|---|---|
| | A | W | P |
| Glycerin | 5.0 | 3.0 | 7.0 |
| CAPB | 4.5 | 6.0 | 2.1 |
| PEG-18 glyceryl oleate/cocoate | 2.5 | 0.7 | 0.5 |
| Disodium cocodiamphoacetate | 1.2 | 1.2 | 0.6 |
| Sodium cocoyl glycinate | -- | -- | 0.7 |
| Decyl glucoside | 1.1 | 1.1 | |
| Lactic acid | 1.0 | 0.4 | 0.01 |

(continued)

| Ingredients/wt% all at actuals | Reference | | |
|---|---|---|---|
| | A | W | P |
| PEG-150 distearate | 0.7 | 0.6 | 0.6 |
| Caprylyl glycol | -- | -- | 0.2 |
| Symguard® CD | 0.5 | 0.5 | 0.5 |
| Glycogen | 0.01 | 0.01 | 0.01 |
| Water and other minors to | 100.0 | 100.0 | 100.0 |
| Viscosity | 3000 to 6000 | 3000 to 6000 | 4000 to 8000 |
| The pH | 3.4 to 3.9 | 3.9 to 4.3 | 6.7 to 7.3 |

Antimicrobial efficacy

The afore mentioned compositions of Table 1 were subjected to an assessment of antimicrobial activity for water miscible compounds using a Time-Kill procedure as per ASTM E2783 - 11. This test method measures the changes of a population of aerobic and anaerobic microorganisms within a specific sampling time when tested against antimicrobial test materials in vitro. The organisms used are standardized as to growth requirements and inoculum preparation and must grow under the conditions of the test. The primary purpose of this test method is to provide a set of standardized conditions and test organisms to facilitate comparative assessments of antimicrobial materials miscible in aqueous systems.

The observations are summarised in Table 2.

Table 2

| Bacteria | Log reduction in cfu/ml | | | |
|---|---|---|---|---|
| | ** | A | W | P |
| L.crispatus | 6.4 | 2.5 | 2.6 | 2.6 |
| G.vaginalis | 6.8 | 6.8 | 6.8 | 6.8 |

All the formulations gave a complete kill of more than 6-log against G.vaginalis but only the formulations of Table 1 showed maximum of 2.6 log reduction against L.crispatus.

The data in table 2 indicates that the compositions in accordance with the invention are selectively efficacious and balance the microbiome of the female intimate region.

Note: ** was a comparative marketed feminine hygiene product

Example 2: Mildness and Barrier properties

Cleanser induced skin damage has been largely attributed to surfactant interaction with stratum corneum proteins and lipids. In-vitro mildness measurement uses model protein (zein) solubility and lipid retention percentage to screen for potential skin damage caused by surfactant / cleanser.

Zein Solubility: Zein is a corn protein with limited water solubility. Zein solubility typically will increase in surfactant solution, which is due to surfactant's ability to denature and to dissolve the otherwise less water soluble zein protein. A higher zein solubility in surfactant / cleanser solution indicates a potentially harsher surfactant / cleanser towards skin, as shown in clinical patch test. [Reference: K.P. Ananth, et.al. "A Novel Technology in Mild and Moisturizing Cleansing Liquids", Cosmetic Dermatology, P 307-316, No. 6, Vol 22, 2009.]

Zein solubility is tested by mixing zein powder with 5% test formulation at room temperature for 30 min. The solubilized zein is separated from the undissolved portion by a 0.45um syringe filter and is diluted by 2% sodium dodecyl sulfate (SDS) solution. UV measurement is performed on the diluted zein solution with 2% SDS. The value reported is calculated as:

$$\text{UV absorbance} = (I_{278,\ sample} - I_{278,\ blank}) * \text{dilution factor} * 2$$

$I_{278,\ sample}$: Intensity reading at wavelength 278 for the zein solution

$I_{278, blank}$: Intensity reading at wavelength 278 for the Blank solution

Lipid Retention test:

Another aspect of surfactant / cleanser damage to skin is due to the surfactant's ability to solubilize lipid components (fatty acid, cholesterol & ceramide) of stratum corneum, which leads to compromise of skin barrier function. Lipid retain test as described below is to characterize the ability of surfactant/cleanser to solubilize lipids. A higher retain % indicates less interaction of surfactant/cleanser with lipid, which in turn suggests a potentially milder product towards skin lipid. [Reference: K.P. Ananth, et.al. "A Novel Technology in Mild and Moisturizing Cleansing Liquids", Cosmetic Dermatology, P 307-316, No. 6, Vol 22, 2009.]

A mixture of Palmitic acid, Stearic acid, Cholesterol and ceramide is casted onto filter paper to mimic the skin lipid layer. A Florescence dye (12NBD Stearate) is added to the lipid mixture. The lipid paper is immersed into 10% test formulation for 30 minutes at room temperature. At the end of 30 minutes, the lipid paper is taken out and is washed under DI water for 30 seconds. The lipid paper was then dried at room temperature. After drying, the lipid paper is immersed into N ml of Isopropyl alcohol (IPA) for around 40 minutes. The florescence intensity of the IPA extraction solution is measured. The lipid retain % is calculated as following:

Retention % = (FL intensity at 530nm of sample lipid paper * *N ml*) / (FL intensity at 530nm of untreated lipid paper * 5ml)

The observations are summarised in Table 3.

Table 3

| Composition | zein UV absorbance | Lipid retention % |
|---|---|---|
| A | 8.89 | 7.42 |
| W | 9.45 | 9.11 |
| P | 8.52 | 8.85 |
| Water | 8.31 | 61.68 |
| Soap/syndet | 30.66 | 1.21 |

The data in Table 3 indicates that the compositions in accordance with the invention are very mild. From above Table, it is shown that prototypes have a zein solubility close to water, which indicate that they are all mild to skin protein.

The term soap/syndet refers to a standard cleanser containing soap and surfactant(s).

Electrical Impedance - Mildness

Electrical impedance is a proxy indicator of change in skin permeability. Measurements of the electrical impedance of the stratum corneum (or of electrical current through the stratum corneum under a fixed applied voltage) are made at time zero and at a set interval after a test formulation is applied to the human cadaver skin; a decrease in skin impedance over this time period indicates that the test formulation has increased the skin permeability (the extent of permeabilization being reported to scale with the extent of impedance increase), in turn suggesting that the test formulation would lead to a potentially higher degree of skin barrier damage.

The impedance reading at each cell at different time points was normalized against the time zero-minute reading of that cell. For each test formulation, the impedance reading of each time point is the average normalized value of the 6 repeat cells at that time point. Note: SDS = sodium dodecyl sulphate.

The observations are shown in Table 4.

Table 4

| Time/minutes | Water | SDS | A | W | P |
|---|---|---|---|---|---|
| 0 | 1.00 | 1.00 | 1.00 | 1.00 | 1.00 |
| 5 | 1.00 | 0.94 | 0.98 | 0.95 | 1.00 |
| 10 | 1.01 | 0.90 | 0.98 | 0.93 | 1.01 |

(continued)

| Time/minutes | Water | SDS | A | W | P |
|---|---|---|---|---|---|
| 15 | 1.03 | 0.86 | 0.97 | 0.91 | 1.02 |
| 30 | 0.96 | 0.76 | 0.97 | 0.89 | 1.02 |

The data indicates that impedance of the skin when treated with compositions of the invention was comparable to that of skin treated with water.

**Claims**

1. An aqueous sulphate-free cleansing composition for female intimate hygiene, the composition comprising:

   a) 1 to 20 wt% of a non-soap surfactant system comprising combination of at least three sulphate-free surfactants selected from glycinates, betaines, alkyl polyglucosides and amphoacetates;
   b) 0.05 to 4 wt% of a prebiotic capable of being broken down by Lactobacilli to lactic acid; and,
   c) 0.001 to 3 wt% lactic acid or a salt thereof added as a postbiotic;

   wherein said composition is free of live bacteria including Lactobacilli.

2. An aqueous composition according to claim 1, wherein when the non-soap surfactant system comprises glycinates, the glycinates are in the range of 0.5 to 5 wt%.

3. An aqueous composition according to claim 1 or 2, wherein when the non-soap surfactant system comprises betaines, the betaines are in the range of 0.5 to 8 wt%.

4. An aqueous composition according to anyone of the preceding claims from 1 to 3, wherein when the non-soap surfactant system comprises alkyl polyglucosides, the alkyl polyglucosides are selected from the group comprising ethers or mixtures of ethers of fatty alcohols comprising from 12 to 44 carbon atoms and of glucose, maltose, sucrose, xylose or fructose, and ethers or mixtures of ethers of fatty alcohols comprising from 12 to 44 carbon atoms and of methylglucose.

5. An aqueous composition according to anyone of the preceding claims from 1 to 4, wherein pH of the composition is from 3.0 to 7.5.

6. An aqueous composition according to anyone of the preceding claims from 1 to 5, wherein the composition comprises a rheological additive selected from the group comprising, polyethylene glycol distearates, acylates, starch, derivatives of starch, carbohydrate gums, cationic polymer, cationic galactomannans, high molecular weight polyethylene glycols, waxes or polymeric thickeners.

7. An aqueous composition according to anyone of the preceding claims from 1 to 6, wherein viscosity of the composition is in the range of 2500 to 10000 cps, wherein the viscosity is determined using a Brookfield V2 viscometer, spindle RTV5, 1 minute, 20rpm, at 30°C.

8. An aqueous composition according to any of the preceding claims 1 to 7, wherein the prebiotic is an oligosaccharide, fructan, sugar alcohol (glycerol or lactitol) or a complex polysaccharide.

9. An aqueous composition according to any of the preceding claims 1 to 8, wherein the composition comprises a preservative selected from sodium benzoate, phenoxyethanol, 4-isopropyl-3-methylphenol, sodium salicylate, parabens, hydantoins, benzyl alcohol, benzoic acid, 1,2-alkanediols, iodopropynyl butylcarbamate (IPBC), 5-chloro-2-methyl-2H-isothiazol-3-one, 2-methyl-2H-isothiazol-3-one, caprylyl glycol, phenylpropanol, o-Cymen-5-ol and decylene glycol or mixtures thereof.

10. An aqueous composition according to any of the preceding claims 1 to 9, wherein the composition comprises 2 to 15 wt% humectant selected from the group consisting of propylene glycol, hexylene glycol, 1,4-dihydroxyhexane, 1,2,6-hexanetriol, sorbitol, butylene glycol, caprylyl glycol, propanediols, such as methyl propane diol, dipropylene glycol, triethylene glycol, glycerin (glycerol), polyethylene glycols, ethoxydiglycol, polyethylene sorbitol, glyceryl caprylate/caprate, and combinations thereof.

11. An aqueous composition according to any of the preceding claims 1 to 10, wherein the composition reduces the count of at least G.vaginalis inhabiting female intimate region by at least 5-log but does not reduce the count of at least L.crispatus by more than 3 log using Time-Kill procedure as per ASTM E2783 - 11.

12. An aqueous composition according to any of the preceding claims 1 to 11, wherein the prebiotic is an oligosaccharide.

13. Non-therapeutic use of an aqueous composition according to any of the preceding claims 1 to 12 for female intimate hygiene.

14. A non-therapeutic method of providing female intimate hygiene comprising a step of contacting a female intimate region with the composition according to any of the preceding claims 1 to 12 for a contact time of at least 30 seconds.

**Patentansprüche**

1. Wässrige sulfatfreie Reinigungszusammensetzung für die Intimhygiene von Frauen, wobei die Zusammensetzung umfasst:

a) 1 bis 20 Gew.-% eines seifenfreien Tensidsystems, das eine Kombination aus mindestens drei sulfatfreien Tensiden umfasst, ausgewählt unter Glycinaten, Betainen, Alkylpolyglucosiden und Amphoacetaten;
b) 0,05 bis 4 Gew.-% eines Präbiotikums, das von Laktobazillen zu Milchsäure abgebaut werden kann; und
c) 0,001 bis 3 Gew.-% Milchsäure oder eines Salzes davon, das als Postbiotikum zugesetzt ist; wobei die Zusammensetzung frei von lebenden Bakterien ist, einschließlich Laktobazillen.

2. Wässrige Zusammensetzung nach Anspruch 1, wobei, wenn das seifenfreie Tensidsystem Glycinate umfasst, die Glycinate in dem Bereich von 0,5 bis 5 Gew.-% vorliegen.

3. Wässrige Zusammensetzung nach Anspruch 1 oder 2, wobei, wenn das seifenfreie Tensidsystem Betaine umfasst, die Betaine in dem Bereich von 0,5 bis 8 Gew.-% vorliegen.

4. Wässrige Zusammensetzung nach irgendeinem der vorhergehenden Ansprüche 1 bis 3, wobei, wenn das seifenfreie Tensidsystem Alkylpolyglucoside umfasst, wobei die Alkylpolyglucoside aus der Gruppe ausgewählt sind, umfassend Ether oder Gemische von Ethern von Fettalkoholen mit 12 bis 44 Kohlenstoffatomen und von Glucose, Maltose, Saccharose, Xylose oder Fructose sowie Ether oder Gemische von Ethern von Fettalkoholen mit 12 bis 44 Kohlenstoffatomen und von Methylglucose.

5. Wässrige Zusammensetzung nach irgendeinem der vorhergehenden Ansprüche 1 bis 4, wobei der pH-Wert der Zusammensetzung zwischen 3,0 und 7,5 liegt.

6. Wässrige Zusammensetzung nach irgendeinem der vorhergehenden Ansprüche 1 bis 5, wobei die Zusammensetzung ein rheologisches Additiv umfasst, das aus der Gruppe ausgewählt ist, umfassend Polyethylenglykoldistearate, Acylate, Stärke, Stärkederivate, Kohlenhydratgummis, kationisches Polymer, kationische Galaktomannane, Polyethylenglykole mit hohem Molekulargewicht, Wachse oder polymere Verdickungsmittel.

7. Wässrige Zusammensetzung nach irgendeinem der vorhergehenden Ansprüche 1 bis 6, wobei die Viskosität der Zusammensetzung im Bereich von 2500 bis 10000 cps liegt, wobei die Viskosität unter Verwendung eines Brookfield V2-Viskosimeters, Spindel RTV5, 1 Minute, 20 U/min, bei 30°C bestimmt wird.

8. Wässrige Zusammensetzung nach einem der vorhergehenden Ansprüche 1 bis 7, wobei das Präbiotikum ein Oligosaccharid, ein Fruktan, ein Zuckeralkohol (Glycerin oder Lactitol) oder ein komplexes Polysaccharid ist.

9. Wässrige Zusammensetzung nach einem der vorhergehenden Ansprüche 1 bis 8, wobei die Zusammensetzung ein Konservierungsmittel umfasst, das ausgewählt ist unter Natriumbenzoat, Phenoxyethanol, 4-Isopropyl-3-methylphenol, Natriumsalicylat, Parabenen, Hydantoinen, Benzylalkohol, Benzoesäure, 1,2,-Alkandiolen, Iodopropynylbutylcarbamat (IPBC), 5-Chlor-2-methyl-2H-isothiazol-3-on, 2-Methyl-2H-isothiazol-3-on, Caprylylglykol, Phenylpropanol, o-Cymen-5-ol und Decylenglykol oder Mischungen davon.

10. Wässrige Zusammensetzung nach einem der vorhergehenden Ansprüche 1 bis 9, wobei die Zusammensetzung 2 bis 15 Gew.-% Feuchthaltemittel umfasst, ausgewählt aus der Gruppe, bestehend aus Propylenglykol, Hexylenglykol, 1,4-Dihydroxyhexan, 1,2,6-Hexantriol, Sorbit, Butylenglykol, Caprylylglykol, Propandiolen, wie Methylpropandiol, Dipropylenglykol, Triethylenglykol, Glycerin (Glycerol), Polyethylenglykolen, Ethoxydiglykol, Polyethylensorbit, Glycerylcaprylat/Caprate und Kombinationen davon.

11. Wässrige Zusammensetzung nach einem der vorhergehenden Ansprüche 1 bis 10, wobei die Zusammensetzung die Anzahl von mindestens G. vaginalis im weiblichen Intimbereich um mindestens 5-log reduziert, jedoch die Anzahl von mindestens L. crispatus unter Verwendung des Time-Kill-Verfahrens gemäß ASTM E2783-11 um nicht mehr als 3-log reduziert.

12. Wässrige Zusammensetzung nach einem der vorhergehenden Ansprüche 1 bis 11, wobei das Präbiotikum ein Oligosaccharid ist.

13. Nicht-therapeutische Verwendung einer wässrigen Zusammensetzung nach einem der vorhergehenden Ansprüche 1 bis 12 für die Intimhygiene von Frauen.

14. Nicht-therapeutisches Verfahren zur Gewährleistung der Intimhygiene bei Frauen, umfassend den Schritt des Inkontaktbringens eines weiblichen Intimbereichs mit der Zusammensetzung nach einem der vorhergehenden Ansprüche 1 bis 12 für eine Kontaktzeit von mindestens 30 Sekunden.

**Revendications**

1. Composition aqueuse de nettoyage sans sulfate pour l'hygiène intime féminine, la composition comprenant :

    a) 1 à 20 % en poids d'un système tensioactif sans savon comprenant une combinaison d'au moins trois tensioactifs sans sulfate choisis parmi les glycinates, les bétaïnes, les alkylpolyglucosides et les amphoacétates ;
    b) 0,05 à 4 % en poids d'un prébiotique pouvant être cassé par les lactobacilles en acide lactique ; et
    c) 0,001 à 3 % en poids d'acide lactique ou d'un sel de celui-ci ajouté en tant que post-biotique ;

    dans laquelle ladite composition est exempte de bactéries vivantes y compris de lactobacilles.

2. Composition aqueuse selon la revendication 1, dans laquelle, lorsque le système tensioactif sans savon comprend des glycinates, les glycinates sont dans la plage allant de 0,5 à 5 % en poids.

3. Composition aqueuse selon la revendication 1 ou 2, dans laquelle, lorsque le système tensioactif sans savon comprend des bétaïnes, les bétaïnes sont dans la plage allant de 0,5 à 8 % en poids.

4. Composition aqueuse selon l'une quelconque des revendications précédentes 1 à 3, dans laquelle, lorsque le système tensioactif sans savon comprend des alkylpolyglucosides, les alkylpolyglucosides sont choisis dans le groupe comprenant les éthers ou des mélanges d'éthers d'alcools gras comprenant de 12 à 44 atomes de carbone et de glucose, maltose, saccharose, xylose ou fructose, et les éthers ou les mélanges d'éthers d'alcools gras comprenant de 12 à 44 atomes de carbone et de méthylglucose.

5. Composition aqueuse selon l'une quelconque des revendications précédentes 1 à 4, dans laquelle le pH de la composition va de 3,0 à 7,5.

6. Composition aqueuse selon l'une quelconque des revendications précédentes 1 à 5, dans laquelle la composition comprend un additif rhéologique choisi dans le groupe comprenant les distéarates de polyéthylène glycol, les acylates, l'amidon, les dérivés d'amidon, les gommes d'hydrate de carbone, un polymère cationique, les galactomannanes cationiques, les polyéthylène glycols de poids moléculaire élevé, les cires ou les épaississants polymères.

7. Composition aqueuse selon l'une quelconque des revendications précédentes 1 à 6, dans laquelle la viscosité de la composition se situe dans la plage de 2500 à 10000 cps, dans laquelle la viscosité est déterminée en utilisant un viscosimètre de Brookfield V2, mobile RTV5, 1 minute, 20 t/min, à 30 °C.

8. Composition aqueuse selon l'une quelconque des revendications précédentes 1 à 7, dans laquelle le prébiotique est un oligosaccharide, un fructane, un alcool de sucre (glycérol ou lactitol) ou un polysaccharide complexe.

9. Composition aqueuse selon l'une quelconque des revendications précédentes 1 à 8, dans laquelle la composition comprend un conservateur choisi parmi le benzoate de sodium, le phénoxyéthanol, le 4-isopropyl-3-méthylphénol, le salicylate de sodium, les parabènes, les hydantoïnes, l'alcool benzylique, l'acide benzoïque, les 1,2-alcanediols, l'iodopropynyl butylcarbamate (IPBC), la 5-chloro-2-méthyl-2H-isothiazol-3-one, la 2-méthyl-2H-isothiazol-3-one, le caprylyl glycol, le phénylpropanol, le o-cymèn-5-ol et le décylène glycol ou des mélanges de ceux-ci.

10. Composition aqueuse selon l'une quelconque des revendications précédentes 1 à 9, dans laquelle la composition comprend 2 à 15 % en poids d'agent hydratant choisi dans le groupe constitué par le propylène glycol, l'hexylène glycol, le 1,4-dihydroxyhexane, le 1,2,6-hexanetriol, le sorbitol, le butylène glycol, le caprylyl glycol, les propanediols, comme le méthylpropane diol, le dipropylène glycol, le triéthylène glycol, la glycérine (le glycérol), les polyéthylène glycols, l'éthoxydiglycol, le polyéthylène sorbitol, le caprylate/caprate de glycéryle et des combinaisons de ceux-ci.

11. Composition aqueuse selon l'une quelconque des revendications précédentes 1 à 10, dans laquelle la composition réduit le nombre d'au moins G. vaginalis colonisant la région intime féminine d'au moins un facteur 5-log mais ne réduit pas le nombre d'au moins L. crispatus de plus de 3 log en utilisant la procédure d'efficacité antimicrobienne (time-kill) telle qu'indiquée dans la norme ASTM E2783-11.

12. Composition aqueuse selon l'une quelconque des revendications précédentes 1 à 11, dans laquelle le prébiotique est un oligosaccharide.

13. Utilisation non thérapeutique d'une composition aqueuse selon l'une quelconque des revendications précédentes 1 à 12 pour l'hygiène intime féminine.

14. Procédé non thérapeutique de fourniture d'une hygiène intime féminine comprenant une étape de mise en contact d'une région intime féminine avec la composition selon l'une quelconque des revendications précédentes 1 à 12 pendant une durée de contact d'au moins 30 secondes.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 20244898 A1
- WO 2017055789 A1
- US 20190262292 A1
- EP 1481666 A1
- WO 2019093060 A
- US 20190274937 A1
- WO 2006015726 A1

**Non-patent literature cited in the description**

- **K.P. ANANTH**. A Novel Technology in Mild and Moisturizing Cleansing Liquids. *Cosmetic Dermatology*, 2009, vol. 22 (6), 307-316